# EUROPÄISCHE PATENTSCHRIFT

(11) **EP 0 387 694 B1**
(45) Veröffentlichungstag und Bekanntmachung des Hinweises auf die Patenterteilung: **31.01.1996**
(21) Anmeldenummer: 90104407.3
(22) Anmeldetag: 08.03.1990
(51) Int. Cl.: A61M 37/00

(54) **Pflaster als therapeutisches System zur Verabreichung von Wirkstoffen an die Haut mit einer abgestuften Wirkstoffabgabe, Verfahren zu seiner Herstellung sowie Verwendung**
Plaster as a therapeutic system for the stepwise release of medicaments to the skin, and process for its manufacture
Pansement thérapeutique pour la libération par paliers d'un médicament sur la peau et son procédé de fabrication

(30) Priorität: 15.03.1989 DE 3908432
(43) Veröffentlichungstag der Anmeldung: 19.09.1990
(73) Patentinhaber: LTS Lohmann Therapie-Systeme GmbH & Co. KG, D-56513 Neuwied (DE)
(72) Erfinder: Hoffmann, Annegrete, D-5450 Neuwied 22 (DE)
(74) Vertreter: Flaccus, Rolf-Dieter, Dr.

(56) Entgegenhaltungen:
- EP-A- 0 249 475
- WO-A-88/01516
- WO-A-89/09080
- US-A- 4 262 003
- US-A- 4 698 062

## Beschreibung

Die Erfindung betrifft ein Pflaster als therapeutisches System zur Verabreichung von Wirkstoffen an die Haut mit einer abgestuften Wirkstoffabgabe, Verfahren zu seiner Herstellung sowie Verwendung für die lokale oder systemische dermale Wirkstoffverabreichung in der Kosmetik.

Therapeutische Vorrichtungen zur gesteuerten Verabreichung von Wirkstoffen werden als therapeutische Systeme bezeichnet (K.Heilmann, Therapeutische Systeme, S. 26, F.Enke Verlag Stuttgart 1984). Danach ist ein solches System eine arzneistoffenthaltende Vorrichtung bzw. eine Darreichungsform, die einen Arzneistoff oder mehrere in vorausbestimmter Rate kontinuierlich über einen festgelegten Zeitraum an einen festgelegten Anwendungsort abgibt.

Bei Anwendung an der Haut können derartige Systeme sowohl topische als auch systemische Wirkung entfalten, und die Vielfalt der auf diese Weise applizierbaren Wirkstoffe und ihre unterschiedlichen chemischen, physikalischen und pharmakologischen Eigenschaften stellen stets neue Anforderungen bei der Herstellung derartiger Systeme. So sind für die dermale Applikation für viele Wirkstoffe schon Problemlösungen gefunden worden, bei denen über die gewünschte Applikationszeit eine kontinuierliche, weitgehend gleichbleibende Freisetzung erreicht wird.

Daneben existieren aber auch therapeutische Erfordernisse, nach denen das Freisetzungsprofil des Wirkstoffes nach einer erhöhten Initialdosis eine niedrigere Erhaltungsdosis aufweisen soll. So ist beispielsweise bei Einschlafmitteln eine erhöhte Zufuhr des Wirkstoffs bei der Schlafinduktion und eine niedrigere Dosis zum Verhindern des Aufwachens in einem längeren anschließenden Zeitraum erwünscht. Auch bei Appetitzüglern soll in den Tagesstunden ein höherer Blutspiegel des Wirkstoffes einstellbar sein als in der Nacht. Dasselbe betrifft auch die Verabreichung von Nikotin zur Raucherentwöhnung. Nicht zuletzt sei erwähnt, daß bei der Bekämpfung von Schmerzattacken nach einer erhöhten Initialdosis eine verminderte Erhaltungsdosis wünschenswert ist.

Ein transdermales therapeutisches System zur Lösung dieses Problems ist schon in der EP-A 0 227 252 beschrieben worden. Dort wird der Wirkstoff in einem Reservoir nur mit soviel Penetrationsbeschleuniger in Kontakt gebracht, daß die beschleunigte Penetration nur während einer definierten Anfangsphase der Applikation aufrechterhalten wird. Nachteilig ist hier, daß für jeden Wirkstoff ein geeigneter Penetrationsbeschleuniger gefunden und bei der Wahl der Reservoirmatrix neben der Diffusionsfähigkeit des Wirkstoffes auch die des Penetrationsbeschleunigers berücksichtigt werden muß.
Letzteres führt in vielen Fällen dazu, daß eine aufwe-dige zusätzliche Steuermembran notwendig wird.

Eine andere Problemlösung wird in der DE-OS 36 42 931 vorgeschlagen. Dort sind mindestens zwei voneinander getrennte Kammern des Pflasters mit unterschiedlichen Wirkstoffkonzentrationen versehen, so daß in der ersten Phase der Applikation die Freigabe des Wirkstoffes aus allen Kammern eine hohe Initialdosis darstellt, während nach Entleerung der Kammern mit niedriger Wirkstoffkonzentration nur noch die Kammern mit höherer Wirkstoffkonzentration zur Freigabe beitragen und somit eine erniedrigte Erhaltungsdosis bewirken. Dieses System ist schon von der Kammerkonstruktion her aufwendig und bedarf bezüglich der verschiedenen Konzentrationseinstellungen in den Kammern besonderer Maßnahmen.

WO-A-88/01516 beschreibt ein Pflaster als transdermales therapeutisches System zur gesteuerten, abgestuften Verabreichung von Wirkstoffen an die Haut mit hoher Initialdosis und niedrigerer Erhaltungsdosis, welches eine der Haut abgewandte Rückschicht, mindestens ein Wirkstoffdepot im Kontakt mit einer die Wirkstoffabgabe steuernden Matrix und eine haftklebende Fixierungseinrichtung für das Pflaster auf der Haut umfaßt, wobei die gesamte Matrix zu Beginn der Applikation wirkstoffhaltig ist und ein oder mehrere räumlich definiert zueinander angeordnete, diskrete Wirkstoffdepots mit einer höheren Wirkstoffkonzentration als in der Reservoirmatrix aufweist.

Ausgehend von diesem Stand der Technik liegt der Erfindung die Aufgabe zu Grunde, ein Pflaster als therapeutisches System zur Verabreichung von Wirkstoffen an die Haut mit einer abgestuften Wirkstoffabgabe bereitzustellen, das die zwingende Anwesenheit eines Penetrationsbeschleunigers vermeidet und in einfacher Weise herstellbar ist.

Die Aufgabe wird erfindungsgemäß überraschenderweise durch ein Pflaster mit hoher Initialdosis und niedrigerer Erhaltungsdosis mit einer der Haut abgewandten Rückschicht, mindestens einem Wirkstoffdepot im Kontakt mit einer die Wirkstoffabgabe steuernden Matrix und einer haftklebenden Fixierungseinrichtung für das Pflaster auf der Haut gelöst, wobei die gesamte Matrix zum Zeitpunkt der Applikation wirkstoffhaltig ist und die Dimensionierung des oder der Wirkstoffdepots sowie die Positionierung des oder der Wirkstoffdepots im Kontakt mit der Matrix so gewählt sind, daß zumindest in einer Richtung der Abstand zwischen Depot und einem die Abgabefläche berührenden Matrixrand größer ist, als der Diffusionsweg mindestens eines Wirkstoffs vom Depot in die Matrix während der Zeitdauer der Applikation und die Menge des Wirkstoffgehaltes in der Matrix zum Zeitpunkt der Applikation so gewählt ist, daß die Zeitdauer der Freigabe bis zur Freisetzung des ursprünglich in der Matrix enthaltenen Wirkstoffs kürzer als die Zeitdauer der Applikation ist
Gegenstand der Erfindung ist somit ein Pflaster als transdermales therapeutisches System zur gesteuerten, abgestuften Verabreichung von Wirkstoffen an die Haut mit hoher Initialdosis und niedrigerer Erhaltungsdosis, das eine der Haut abgewandte Rückschicht, mindestens ein Wirkstoffdepot im Kontakt mit einer die Wirkstoffabgabe steuernden Matrix und eine haftklebende Fixierungseinrichtung für das Pflaster auf der Haut umfaßt, wobei die gesamte Matrix zu Beginn der Applikation wirkstoffhaltig ist, welches dadurch gekennzeichnet ist, daß die Dimensionierung und die Positionierung des oder der Wirkstoffdepots in Kontakt mit der Matrix so gewählt sind, daß zumindest in einer Richtung der Abstand zwischen Depot und einem die Abgabefläche berührenden Matrixrand größer ist als der der Diffusionsweg mindestens eines Wirkstoffes vom Depot in die Matrix während der Zeitdauer der Applikation und die Menge des Wirkstoffgehalts in der Matrix zum Zeitpunkt der Applikation so gewählt ist, daß die Zeitdauer der Freigabe bis zur Freisetzung des ursprünglich in der Matrix enthaltenen Wirkstoffs kürzer als die Zeitdauer der Applikation ist.

Der prinzipielle Aufbau des erfindungsgemäßen Pflasters ist aus DE-OS 21 35 533 bekannt, ohne daß dort aber die erfindungsgemäßen konstruktiven Merkmale für die angestrebte Abänderung des Freisetzungsprofils erkannt worden wären.

Ein wesentliches Merkmal des erfindungsgemäßen Pflasters ist ein Wirkstoffgehalt der Matrix zum Zeitpunkt der Applikation. Dies kann auf zwei Wegen erreicht werden. Einmal kann die Matrix schon bei der Herstellung des Pflasters mit Wirkstoff beladen werden, oder die Wirkstoffbeladung erfolgt zum anderen nach der Herstellung des Pflasters durch Diffusion von Wirkstoff aus dem Wirkstoffdepot in die bis dahin leere Matrix. Der letztgenannte Vorgang läßt sich durch Temperaturerhöhung beschleunigen und kann Stunden bis Tage dauern. Damit kann während der Anfangsphase der Applikation über die gesamte Freigabefläche der Matrix Wirkstoff austreten (entspricht hoher Initialdosis), was zu einer Verarmung an Wirkstoff in den angrenzenden Schichten führt. Diese Verarmung kann nur durch Nachlieferung von Wirkstoff aus dem Wirkstoffdepot ausgeglichen werden. Ist nun der Abstand zwischen Depot und Entleerungszonen so groß, daß er während der Applikationsdauer aufgrund des Diffusionsvermögens des Wirkstoffes nicht überwunden werden kann, so kommt die Wirkstoffabgabe in diesen Bezirken zum Stillstand. Nur die Anteile der Freigabefläche der Matrix setzen noch Wirkstoff frei, die durch Nachdiffusion aus dem Depot versorgt werden können (entspricht verringerter Erhaltungsdosis). Die geometrischen Verhältnisse im Pflaster werden demnach im wesentlichen durch das angestrebte Freisetzungsprofil, den zu verabreichenden Wirkstoff, die Auswahl der Matrix und die Dimensionierung sowie die Positionierung der Depots bestimmt.

Das Wirkstoffdepot mit einem oder mehreren Wirkstoffen kann aus reinem Wirkstoff bestehen, der fest oder fleißfähig sein kann, aber auch inerte Hilfsstoffe aufweisen. Unter "inert" soll hier verstanden werden, daß Wirkstoff und Hilfsstoff nicht miteinander reagieren. Zu diesen dem Fachmann bekannten inerten Hilfsstoffen gehören beispielsweise Lösemittel, Füllstoffe, Stabilisatoren, Stützmaterialien, Trägerstoffe und gegebenenfalls auch diffusions- und penetrationsregulierende Zusätze.

Als Wirkstoffe können alle transdermal anwendbaren Wirkstoffe eingesetzt werden, für deren Applikation eine hohe Initialdosis angezeigt ist. Hierzu gehören beispielsweise Wirkstoffe aus der Gruppe der Analgetika, Antiemetika, Antiadiposita, Antiphlogistika, Antispasmolytika und Antiangina-Wirkstoffe. Auch das Nikotin gehört als Wirkstoff zur Raucherentwöhnung dazu.

Die Summe der Wirkstoffmengen im Depot und in der Matrix liegt vorteilhafterweise beim bis zum 20-fachen der therapeutisch notwendigen Menge.

Bevorzugt ist die Matrix schichtweise und/oder schichtförmig aufgebaut, wobei die Schichten gleich oder unterschiedlich sein können. Die Matrix kann haftklebend sein, was durch Einsatz geeigneter Polymermaterialien, wie beispielsweise Kautschuk, kautschukähnliche synthetische Homo-, Co- oder Blockpolymere, Poly(meth)acrylate und deren Copolymerisate, Polyurethane und Silicone erreicht werden kann. Grundsätzlich kommen alle Polymeren infrage, die bei der Herstellung von Haftklebern eingesetzt werden und physiologisch unbedenklich sind. Es kann günstig sein, wenn die Matrix haftklebend ist, da dadurch die Notwendigkeit einer separaten haftklebenden Fixierungseinrichtung für die Vorrichtung vermieden wird. Im Falle einer nicht haftklebenden Matrix werden geeignete Polymermaterialien eingesetzt, die beispielsweise aus der Gruppe der Poly(meth)acrylate, Polyvinylpyrrolidone, Ethylcellulose, Hydroxypropylcellulosen, Hydroxypropylmethylcellulosephthalate, Polyvinylalkohole bzw. deren Copolymerisate mit Vinyllaurat oder Maleinsäure, Vinylacetate bzw. deren Copolymerisate mit Vinyllaurat oder Maleinsäure, Polyvinylether, Butylkautschuke und Polycaprolactame ausgewählt sind.

Das Wirkstoffdepot kann in Form einer Schicht und/oder in sich schichtweise aufgebaut sein. Die Schichtform des Wirkstoffdepots ist aus fertigungstechnischen Gründen immer dann bevorzugt, wenn es gelingt, die Konzentration des Wirkstoffes in der Schicht den Erfordernissen entsprechend einzustellen und den notwendigen Abstand zum Matrixrand einzuhalten. Ein schichtweiser Aufbau des Wirkstoffdepots in sich, wobei das Depot als solches nicht schichtförmig zu sein braucht, ist dann angezeigt, wenn sich eine direkte Mischung von wirkstoffhaltigen Depotanteilen mit notwendigen oder wünschenswerten inerten Hilfsstoffen verbietet oder sich fertigungstechnische Vorteile ergeben.

Beim Aufbau des Pflasters kann beispielsweise mindestens ein Wirkstoffdepot zwischen einer rückseitigen Matrixschicht und einer hautseitigen Matrixschicht eingebracht werden, wobei das Dickenverhältnis der Matrixschichten bevorzugt im Bereich von 1 : 1 bis 1 : 20 und besonders bevorzugt im Bereich von 1 : 1 bis 1 : 5 liegt.

Bei einer weiteren vorteilhaften Ausgestaltung des erfindungsgemäßen Pflasters kann das Wirkstoffdepot zwischen Matrix und Rückschicht angeordnet sein, was sich beispielsweise besonders für feste, in Form von Körpern aufbringbare Wirkstoffdepots eignet.

Ist die Matrix nicht selbstklebend, so kann auf der hautseitigen Fläche der Vorrichtung eine Haftklebeschicht vorgesehen werden. Auch der innere Zusammenhalt der Vorrichtung kann in diesem Falle durch zusätzlich haftklebende Zwischenschichten bewerkstelligt werden. Bei einer bevorzugten Ausführungsform der Erfindung kann die Fixierungseinrichtung durch in die Matrix eingebettete Haftklebeabschnitte oder die Freigabefläche umschließende Haftkleberänder gebildet sein. Die Haftklebeschichten können auch wirkstoffhaltig sein und tragen damit zur Erhöhung der Initialdosis bei.

Die Rückschicht dient zum Schutz und/oder zur mechanischen Stabilisierung der Vorrichtung. Sie kann aus flexiblem oder nicht flexiblem Material bestehen und ein- oder mehrschichtig ausgestattet sein.

Substanzen für ihre Herstellung sind polymere Substanzen, wie beispielsweise Polyethylen, Polypropylen, Polyester und Polyamide. Als weitere Materialien können auch Metallfolien, wie Aluminiumfolie, allein oder mit einem polymeren Substrat beschichtet, angewandt werden. Auch textile Flächengebilde können verwendet werden, wenn sie das Entweichen von Bestandteilen der Vorrichtung zu verhindern vermögen. Besonders bewährt haben sich metallbedampfte Polymerfolien.

In üblicher Weise ist es möglich, eine ablösbare Schutzschicht für die der Haut zugewandten Flächen des Pflasters vorzusehen, die eine unliebsame Verschmutzung und das vorzeitige Entweichen von Bestandteilen des Pflasters verhindert. Sie wird erst unmittelbar vor der Applikation abgelöst. Für ihre Herstellung eignen sich im Prinzip dieselben Materialien wie für die Rückschicht, vorausgesetzt, daß sie ablösbar sind, wie z.B. durch eine Silikonbehandlung. Andere ablösbare Schutzschichten sind beispielsweise Polytetrafluorethylen, behandeltes Papier, Cellophan und Polyvinylchlorid. Natürlich kann die Schutzschicht mit einer Anfaßhilfe versehen sein, um das Abziehen vom Pflaster zu erleichtern.

Ein bevorzugtes Verfahren zur Herstellung des erfindungsgemäßen Pflasters besteht in der in situ-Erzeugung des Wirkstoffdepots. Dabei wird das Depot unmittelbar an der Kontaktstelle zu der Matix aus den Depotkomponenten gebildet. Im übrigen werden die Schichten der Vorrichtung durch Druck-und/oder Wärmeanwendung zusammengefügt. Das Depot kann auch durch Druckanwendung in die Matrix eingebracht werden, beispielsweise durch Injektion einer vorbestimmten Menge oder Eindrücken eines Depotkörpers in eine weiche Matrix. Bei einem bevorzugten Verfahren wird das Wirkstoffdepot zwischen zwei Matrixschichten, die gleich oder unterschiedlich sein können, eingebracht.

Vorzugsweise wird mindestens ein Teil des Pflasters aus einer Lösung, einer Dispersion, einer Schmelze oder durch Aufstreuen von Partikelchen hergestellt. Die so erhaltenen flächenförmigen Zwischenprodukte werden in kleinere Einheiten verteilt, deren Dimensionierung und Formgebung von den therapeutischen Erfordernissen bestimmt werden.

Das erfindungsgemäße Pflaster eignet sich besonders für die lokale und systemische dermale Wirkstoffverabreichung in der Human- oder Tiermedizin oder läßt sich auch in der Kosmetik einsetzen.

Nachfolgend soll die Erfindung anhand von Figuren, in denen unter anderem der schematische nicht maßstabsgetreue Aufbau von erfindungsgemäßen Pflastern dargestellt ist, erläutert werden. Dabei zeigt:
- Figur 1a: einen Schnitt durch ein Pflaster nach dem Stand Technik,
- Figur 1b: eine grafische Darstellung des zum Pfaster nach Fig. 1a gehörenden in vivo-Freisetzungsprofils,
- Figur 2a: einen Schnitt durch eine Ausführungsform des erfindungsgemäßen Pflasters,
- Figur 2b: eine grafische Darstellung des zum Pflaster nach Fig. 2a gehörenden in vivo-Freisetzungsprofils,
- Figur 3a: einen Schnitt durch eine weitere Ausführungsform des erfindungsgemäßen Pflasters,
- Figur 3b: eine grafische Darstellung des zum Pflaster nach Fig. 3a gehörenden in vivo-Freisetzungsprofils,
- Figur 4: einen Schnitt durch eine weitere bevorzugte Ausführungsform des erfindungsgemäßen Pflasters,
- Figur 5: einen Schnitt durch eine weitere bevorzugte Ausführungsform des erfindungsgemäßen Pflasters,
- Figur 6: einen Schnitt durch eine Ausführungsform des erfindungsgemäßen Pflasters mit mehreren Wirkstoffdepots und
- Figur 7: einen Schnitt durch eine Ausführungsform des erfindungsgemäßen Pflasters mit einer Vielzahl von Wirkstoffdepots.

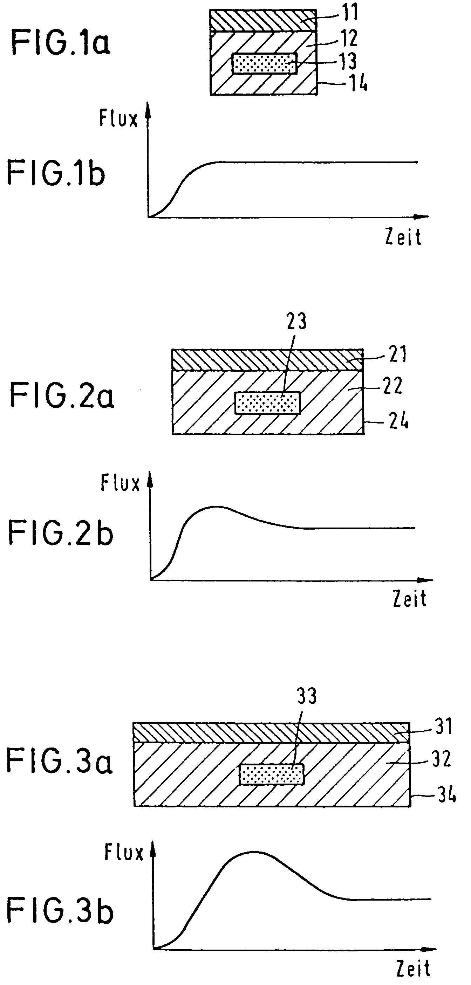

In Figur 1a ist der schematische Schnitt durch ein Pflaster nach dem Stand der Technik dargestellt, wobei in eine haftklebende Matrix (12), die durch eine Rückschicht (11) abgedeckt ist, ein Wirkstoffdepot (13) eingebettet ist. Der Abstand des Wirkstoffdepots (13) vom Matrixrand (14) ist so gering, daß der Wirkstoff während der Applikation durch Diffusion die sich entleerenden Bezirke der Matrix wieder auffüllen kann. Somit tritt während der gesamten Applikationsdauer über die gesamte Freigabefläche Wirkstoff aus. Die Figur 1b gibt das dazugehörende in vivo-Freisetzungsprofil wieder, wobei der Flux gegen die Zeit aufgetragen ist. Es ist zu erkennen, daß nach Erreichen des Maximums der Flux über die restliche Zeit ohne Abstufung weitgehend auf diesem Niveau verbleibt. Nach diesem Stand der Technik wird daher über die gewünschte Zeit ein weitgehend konstanter Flux erhalten.

Der schematische Schnitt durch eine Ausführungsform des erfindungsgemäßen Pflasters in Figur 2a mit der Rückschicht (21), der haftklebenden Matrix (22), dem eingebetteten Depot (23) und dem Matrixrand (24) zeigt einen gegenüber Figur 1a wesentlich vergrößerten Abstand zwischen Depot (23) und Matrixrand (24). Bei der richtigen auf den fraglichen Wirkstoff abgestimmten Wahl der Matrix (22) vermag der Wirkstoff nach Freisetzung des ursprünglich in der Matrix enthaltenen Wirkstoffes im Laufe der weiteren Applikation aufgrund der geometrischen Verhältnisse nicht mehr in die vom Depot (23) entfernteren Bezirke der Matrix (22) nachzudiffundieren. Wie das dazugehörende in vivo-Freisetzungsprofil in Fig. 2b zeigt, sinkt der Flux nach Überschreiten eines Maximums auf einen weitgehend konstanten niedrigeren Wert ab. Damit sind generell nach erhöhten Initialdosen niedrigere Erhaltungsdosen einstellbar.

Fig. 3a stellt den schematischen Schnitt einer weiteren Ausführungsform des erfindungsgemäßen Pflasters mit der Rückschicht (31), der haftklebenden Matrix (32), dem eingebetteten Depot (33) und dem Matrixrand (34) dar. Hier ist der Abstand zwischen Depot (33) und Matrixrand (34) gegenüber der Ausführungsform in Fig. 2a weiter vergrößert, so daß noch größere Bezirke der Matrix (32) während der Applikation nicht mehr mit Wirkstoff versorgt werden können. Da die Gesamtfreigabefläche aber deutlich größer ist als in Fig. 2a, stellt sich gemäß Fig. 3b, in der der dazugehörende in vivo-Flux aufgetragen ist, ein größeres Anfangsmaximun ein, daß dann im Laufe der Applikation auf etwa denselben Wert wie in Fig. 2b absinkt.

Die Positionierung des Depots in der Matrix kann symmetrisch, aber auch asymmetrisch erfolgen, wobei immer gewährleistet sein muß, daß die Entfernung zwischen Depot und Freigabefläche durch Diffusion der Wirkstoffe im Sinne einer gezielten Freigabe überbrückt werden kann. Natürlich spielt dabei das gewünschte Freisetzungsprofil eine entscheidende Rolle. Die günstigste Entfernung kann in manchen Fällen berechnet oder muß in vielen Fällen experimentell ermittelt werden.

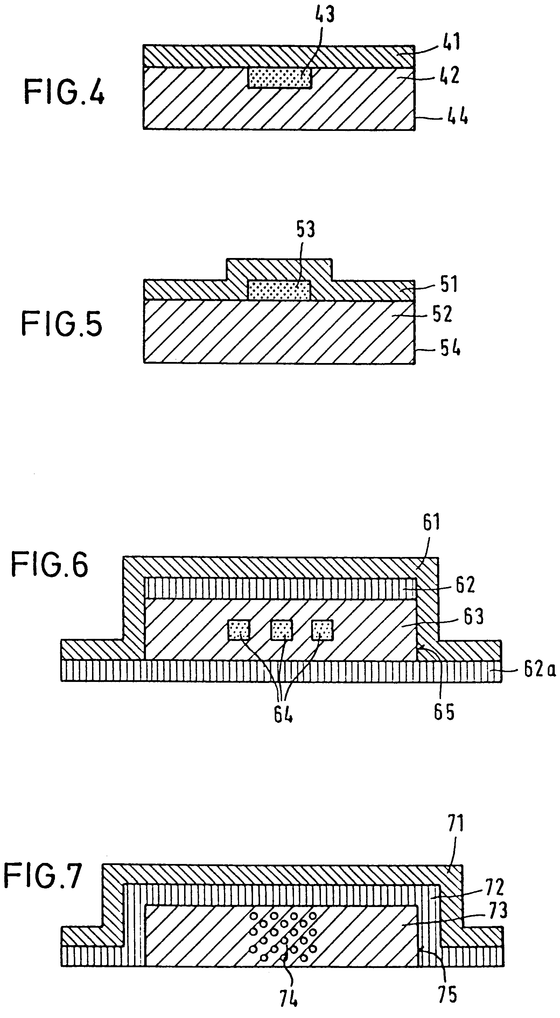

In Figur 4, die den schematischen Schnitt einer weiteren Ausführungsform des erfindungsgemäßen Pflasters zeigt, liegt die Besonderheit in der Positionierung des Depots (43), das nur zum Teil in die haftklebende Matrix (42) eingebettet ist und mit einer Seite fit der Rückschicht (41) in Kontakt steht. Der Abstand des Depots (43) vom Matrixrand (44) entspricht dem in der Fig. 3a, doch beeinflußt der vergrößerte Abstand zwischen Depot (43) und der Freigabefläche die Wahl der für diese Ausführungsform geeigneten Wirkstoffe, wobei diese Ausführungsform fertigungstechnische Vorteile haben kann.

Wie in Figur 5 zu erkennen ist, in der eine weitere Ausführungsform des erfindungsgemäßen Pflasters schematisch im Schnitt wiedergegeben ist, kann das Depot (53) auch auf die haftklebende Matrix (52) aufgesetzt sein, wobei es im übrigen von der Rückschicht (51) umschlossen ist. Für den Abstand zwischen Depot (53) und Matrixrand (54) und jenem zwischen Depot und Freigabefläche gilt das zu Fig. 4 Ausgeführte. Auch hier können außerdem fertigungstechnische Vorteile für die Wahl einer derartigen Ausführungsform sprechen.

Weiterhin ist in Fig. 6 eine Ausführungsform des erfindungsgemäßen Pflasters schematisch im Schnitt abgebildet, bei der in einer nichtklebenden Matrix (63) drei voneinander getrennte Depots (64) eingebettet sind. Die Matrix (63) ist mit der Rückschicht (61) durch eine haftklebende Zwischenschicht (62) verbunden, wobei die Rückschicht (61) auch den Matrixrand (65) abdeckt und einen zur Haut parallelen überstehenden Rand bildet. Die Fixierung des Pflasters auf der Haut wird durch die wirkstoffdurchlässige Haftklebeschicht (62a) bewerkstelligt, die sich über die gesamte freie Fläche der Matrix (63) und die überstehenden Ränder der Rückschicht (61) erstreckt.

Die Ausgestaltung des Pflasters mit mehreren Depots zeigt eine weitere Beeinflussungsmöglichkeit des Freisetzungsprofils auf. Bei gleichem Gesamtvolumen der Depots ermöglicht die räumliche Vereinzelung von Depotteilen im Vergleich zu einem gleichvolumigen Einzeldepot das Nachliefern von Wirkstoff durch Diffusion in größere Bezirke der Matrix. Die erfindungsgemäße Bedingung bezüglich des Abstandes vom Depot zum Pflasterrand muß dabei aber erfüllt bleiben. Selbstverständlich kann auch der Abstand zwischen mindestens zwei Depotteilen so groß sein, daß er den während der Zeitdauer der Applikation möglichen Diffusionsweg mindestens eines Wirkstoffs vom Depotteil in die Matrix übersteigt.

Bezüglich einer weiteren Aufgliederung des Depots gibt Figur 7 ein Beispiel, in dem ein schematischer Schnitt durch eine weitere Ausführungsform des erfindungsgemäßen Pflasters wiedergegeben ist. Die nichtklebende Matrix (73) ist mittig von vielen kleinen Depots (74) durchsetzt, wobei diese feste oder fließfähige Körper oder auch Mikrokapseln darstellen können. Die Matrix (73) ist bis auf die der Haut zugewandten Fläche über eine Haftklebeschicht (72) mit der Rückschicht (71) verbunden, dabei ist auch der Matrixrand (75) abgedeckt. Die Haftklebeschicht (72) erstreckt sich auch über die überstehenden Ränder der Rückschicht (71), womit die Fixierungseinrichtung zur Haut gebildet wird, da die unmittelbar mit der Haut in Kontakt stehende Fläche der Matrix (73) nichtklebend ist.

In allen Figuren mit Schnittzeichnungen durch Pflasterausführungen sind die Schutzschichten für die der Haut zugewandten Flächen der Übersichtlichkeit halber nicht mit eingezeichnet. Selbstverständlich sind sie ein notwendiger Bestandteil der erfindungsgemäßen Pflaster. Die Dimensionierung der Pflaster hinsichtlich Kontur und Größe kann in weiten Grenzen variiert werden, wobei vor allem die therapeutischen und die vom Applikationort bestimmten Erfordernisse, aber auch Gesichtspunkte der Handhabbarkeit zu berücksichtigen sind.

## Patentansprüche (Patentansprüche für folgende(n) Vertragsstaat(en): AT, BE, CH, DE, DK, FR, GB, IT, LI, LU, NL, SE)

1. Pflaster als transdermales therapeutisches System zur gesteuerten, abgestuften Verabreichung von Wirkstoffen an die Haut mit hoher Initialdosis und niedrigerer Erhaltungsdosis, das eine der Haut abgewandte Rückschicht, mindestens ein Wirkstoffdepot im Kontakt mit einer die Wirkstoffabgabe steuernden Matrix und eine haftklebende Fixierungseinrichtung für das Pflaster auf der Haut umfaßt, wobei die gesamte Matrix zu Beginn der Applikation wirkstoffhaltig ist, dadurch gekennzeichnet, daß die Dimensionierung und die Positionierung des oder der Wirkstoffdepots in Kontakt mit der Matrix so gewählt sind, daß zumindest in einer Richtung der Abstand zwischen Depot und einem die Abgabefläche berührenden Matrixrand größer ist als der Diffusionsweg mindestens eines Wirkstoffes vom Depot in die Matrix während der Zeitdauer der Applikation und die Menge des Wirkstoffgehalts in der Matrix zum Zeitpunkt der Applikation so gewählt ist, daß die Zeitdauer der Freigabe bis zur Freisetzung des ursprünglich in der Matrix enthaltenen Wirkstoffs kürzer als die Zeitdauer der Applikation ist.

2. Pflaster nach Anspruch 1, dadurch gekennzeichnet, daß mindestens ein Wirkstoffdepot ausschließlich Wirkstoff(e) aufweist.

3. Pflaster nach Anspruch 1, dadurch gekennzeichnet, daß mindestens ein Wirkstoffdepot mindestens einen inerten Hilfsstoff aufweist.

4. Pflaster nach Ansprüchen 1 bis 3, dadurch gekennzeichnet, daß mindestens ein Wirkstoffdepot in fester oder fließfähiger Form vorliegt.

5. Pflaster nach einem der vorangehenden Ansprüche, dadurch gekennzeichnet, daß es mindestens teilweise schichtweise und/oder schichtförmig aufgebaut ist.

6. Pflaster nach Anspruch 5, dadurch gekennzeichnet, daß die Matrix aus mindestens zwei Schichten besteht, wobei bevorzugt zwischen einer rückseitigen Matrixschicht und einer hautseitigen Matrixschicht mindestens ein Wirkstoffdepot eingebracht ist und das Dickenverhältnis der Matrixschichten bevorzugt im Bereich von 1 : 1 bis 1 : 20 und besonders bevorzugt im Bereich von 1 : 1 bis 1 : 5 liegt.

7. Pflaster nach Anspruch 5, dadurch gekennzeichnet, daß mindestens ein Wirkstoffdepot schichtweise und/oder schichtförmig ausgebildet ist.

8. Pflaster nach einem der vorangehenden Ansprüche, dadurch gekennzeichnet, daß die Matrix haftklebend ist.

9. Pflaster nach den Ansprüchen 1 bis 7, dadurch gekennzeichnet, daß die Matrix oder mindestens eine Matrixschicht mindestens einseitig haftklebende Einrichtungen aufweist.

10. Pflaster nach den Ansprüchen 1 bis 7 und 9, dadurch gekennzeichnet, daß die Fixierungseinrichtung in der Matrix eingebettete Haftklebstoffabschnitte sind.

11. Pflaster nach den Ansprüchen 1 bis 5 und 7 bis 10, dadurch gekennzeichnet, daß mindestens ein Wirkstoffdepot zwischen Matrix und Rückschicht angeordnet ist.

12. Pflaster nach einem der vorangehenden Ansprüche, dadurch gekennzeichnet, daß es eine ablösbare Schutzschicht für die der Haut zugewandten Flächen aufweist.

13. Pflaster nach einem der vorangehenden Ansprüche, dadurch gekennzeichnet, daß es bis zum 20-fachen der therapeutisch notwendigen Wirkstoffmenge enthält.

14. Verfahren zur Herstellung eines Pflasters als transdermales therapeutisches System zur gesteuerten, abgestuften Verabreichung von Wirkstoffen an die Haut nach den Ansprüchen 1 bis 13, dadurch gekennzeichnet, daß das Wirkstoffdepot bei der Herstellung des Pflasters in situ durch Vereinigung von Depotkomponenten herstellt wird.

15. Verfahren nach Ansprüchen 5, 6 und 7, dadurch gekennzeichnet, daß die Schichten durch Druck- und/oder Wärmeanwendung zusammengefügt werden.

16. Verfahren nach einem der vorangehenden Ansprüche, dadurch gekennzeichnet, daß das Wirkstoffdepot durch Druckanwendung in die Matrix eingebracht wird.

17. Verfahren nach einem der vorangehenden Ansprüche, dadurch gekennzeichnet, daß Wirkstoff bei der Herstellung des Pflasters in die Matrix eingebracht wird.

18. Verfahren nach Ansprüchen 1 bis 16, dadurch gekennzeichnet, daß der Wirkstoffgehalt in der Matrix nach der Herstellung des Pflasters durch Diffusion von Wirkstoff aus dem/den Wirkstoffdepot(s) eingestellt wird.

19. Verfahren nach einem der vorangehenden Ansprüche, dadurch gekennzeichnet, daß mindestens ein Teil des Pflasters aus einer Lösung, einer Dispersion, einer Schmelze oder durch Aufstreuen von Partikelchen hergestellt wird.

20. Verwendung des Pflasters nach einem der Ansprüche 1 bis 12 für die lokale oder systemische dermale Wirkstoffverabreichung in der Kosmetik.

## Patentansprüche (Patentansprüche für folgende(n) Vertragsstaat(en): GR, ES)

1. Verfahren zur Herstellung eines Pflasters als transdermales therapeutisches System zur gesteuerten, abgestuften Verabreichung von Wirkstoffen an die Haut mit einer hohen Initialdosis und niedrigeren Erhaltungsdosis, das eine der Haut abgewandte Rückschicht, mindestens ein Wirkstoffdepot im Kontakt mit einer die Wirkstoffabgabe steuernden Matrix und eine haftklebende Fixierungseinrichtung für das Pflaster auf der Haut aufweist, wobei die gesamte Matrix zu Beginn der Applikation wirkstoffhaltig ist, dadurch gekennzeichnet, daß man das oder die Wirkstoffdepots in Kontakt mit der Matrix derart dimensioniert und anordnet, daß zumindest in einer Richtung der Abstand zwischen Depot und einem die Abgabefläche berührenden Matrixrand größer ist als der Diffusionsweg mindestens eines Wirkstoffs vom Depot in die Matrix während der Zeitdauer der Applikation und die Menge des Wirkstoffgehalts in der Matrix zum Zeitpunkt der Applikation so gewählt ist, daß die Zeitdauer der Freigabe bis zur Freisetzung des ursprünglich in der Matrix enthaltenen Wirkstoffs kürzer als die Zeitdauer der Applikation ist.

2. Verfahren nach Anspruch 1, dadurch gekennzeichnet, daß mindestens ein Wirkstoffdepot verwendet wird, welches ausschließlich Wirkstoff(e) enthält.

3. Verfahren nach Anspruch 1, dadurch gekennzeichnet, daß mindestens ein Wirkstoffdepot verwendet wird, welches mindestens einen inerten Hilfsstoff enthält.

4. Verfahren nach Ansprüchen 1 bis 3, dadurch gekennzeichnet, daß mindestens ein Wirkstoffdepot verwendet wird, welches in fester oder fließfähiger Form vorliegt.

5. Verfahren nach einem der vorangehenden Ansprüche, dadurch gekennzeichnet, daß seine Bestandteile wenigstens teilweise schichtförmig aufeinander angeordnet werden.

6. Verfahren nach Anspruch 5, dadurch gekennzeichnet, daß eine aus mindestens zwei Schichten bestehende Matrix verwendet wird, wobei bevorzugt zwischen einer rückseitigen Matrixschicht und einer hautseitigen Matrixschicht mindestens ein Wirkstoffdepot eingebracht ist und das Dickenverhältnis der Matrixschichten bevorzugt im Bereich von 1 : 1 bis 1 : 20 und besonders bevorzugt im Bereich von 1 : 1 bis 1 : 5 liegt.

7. Verfahren nach Anspruch 5, dadurch gekennzeichnet, daß mindestens ein schichtweise und/oder schichtförmig ausgebildetes Wirkstoffdepot verwendet wird.

8. Verfahren nach dem Ansprüchen 1 bis 7, dadurch gekennzeichnet, daß eine haftklebende Matrix verwendet wird.

9. Verfahren nach den Ansprüchen 1 bis 7, dadurch gekennzeichnet, daß die verwendete Matrix oder mindestens eine ihrer Schichten mindestens einseitig haftklebende Einrichtungen aufweist.

10. Verfahren nach den Ansprüchen 1 bis 7 und 9, dadurch gekennzeichnet, daß als Fixierungseinrichtungen in der Matrix eingebettete Haftklebstoffabschnitte verwendet werden.

11. Verfahren nach den Ansprüchen 1 bis 5 und 7 bis 10, dadurch gekennzeichnet, daß wenigstens ein Wirkstoffdepot zwischen Matrix und Rückschicht angeordnet wird.

12. Verfahren nach den Ansprüchen 1 bis 11, dadurch gekennzeichnet, daß auf die der Haut zugewandten Flächen eine ablösbare Schutzschicht aufgebracht wird.

13. Verfahren nach den Ansprüchen 1 bis 12, dadurch gekennzeichnet, daß bis zum 20-fachen der therapeutisch notwendigen Wirkstoffmenge eingearbeitet werden.

14. Verfahren nach den Ansprüchen 1 bis 13, dadurch gekennzeichnet, daß das Wirkstoffdepot bei der Herstellung des Pflasters in situ durch Vereinigung von Depotkomponenten hergestellt wird.

15. Verfahren nach Ansprüchen 5, 6 und 7, dadurch gekennzeichnet, daß die Schichten durch Druck- und/oder Wärmeanwendung zusammengefügt werden.

16. Verfahren nach einem der vorangehenden Ansprüche, dadurch gekennzeichnet, daß das Wirkstoffdepot durch Druckanwendung in die Matrix eingebracht wird.

17. Verfahren nach einem der vorangehenden Ansprüche, dadurch gekennzeichnet, daß Wirkstoff bei der Herstellung des Pflasters in die Matrix eingebracht wird.

18. Verfahren nach Ansprüchen 1 bis 16, dadurch gekennzeichnet, daß der Wirkstoffgehalt in der Matrix nach der Herstellung des Pflasters durch Diffusion von Wirkstoff aus dem/den Wirkstoffdepot(s) eingestellt wird.

19. Verfahren nach einem der vorangehenden Ansprüche, dadurch gekennzeichnet, daß mindestens ein Teil des Pflasters aus einer Lösung, einer Dispersion, einer Schmelze oder durch Aufstreuen von Partikelchen hergestellt wird.

20. Verwendung des Pflasters, hergestellt nach einem der Ansprüche 1 bis 19 für die lokale oder systemische dermale Wirkstoffverabreichung in der Kosmetik.

## Claims (Claims for the following Contracting State(s): AT, BE, CH, DE, DK, FR, GB, IT, LI, LU, NL, SE)

1. A plaster as transdermal therapeutic system for the controlled and graduated administration of active substances to the skin, with a high initial dosage and lower maintenance dosage, comprising a backing layer averted from the skin, at least one active substance depot in contact with a matrix controlling the active substance release, and a pressure-sensitive adhesive fixing device to secure the plaster to the skin, whereby the complete matrix at the beginning of application comprises active substances, characterized in that the dimensions and position of the active substance depot or depots in contact with the matrix are chosen in such a way that, at least in one direction, the space between depot and a matrix edge which contacts the releasing surface is larger than the diffusion path of at least one active substance from the depot into the matrix during the course of application, and that the amount of active substance contained in the matrix is, at the time of application, chosen such that the duration of release until that point in time when the active substance originally contained in the matrix is released is shorter than the duration of application.

2. The plaster according to claim 1, characterized in that at least one active substance depot exclusively comprises active substance or substances.

3. The plaster according to claim 1, characterized in that at least one active substance depot comprises at least one inert auxiliary agent.

4. The plaster according to claims 1 to 3, characterized in that at least one active substance depot is present in solid or flowable form.

5. The plaster according to any one of the preceding claims, characterized in that it is built up at least partially in layers and/or in the form of a layer or laminate.

6. The plaster according to claim 5, characterized in that the matrix consists of at least two layers, whereby preferably at least one active substance depot is incorporated between a back-side matrix layer and a matrix layer facing the skin, and that the thickness ratio of the matrix layers preferably is in the range of 1:1 to 1:20, particularly preferred in the range of 1:1 to 1:5.

7. The plaster according to claim 5, characterized in that at least one active substance depot is formed in layers and/or in the form of a layer or laminate.

8. The plaster according to any one of the preceding claims, characterized in that the matrix is pressure-sensitive adhesive.

9. The plaster according to claims 1 to 7, characterized in that the matrix or at least one matrix layer exhibits pressure-sensitive adhesive devices on at least one side.

10. The plaster according to claims 1 to 7 and 9, characterized in that pressure-sensitive adhesive portions em bedded in the matrix form the fixing device.

11. The plaster according to claims 1 to 5 and 7 to 10, characterized in that at least one active substance depot is positioned between matrix and backing layer.

12. The plaster according to any one of the preceding claims, characterized in that it exhibits a removable protective layer for those surfaces facing the skin.

13. The plaster according to any one of the preceding claims, characterized in that it comprises an amount of active substance amounting to up to 20 times the therapeutically required amount.

14. A process for the production of a plaster as transdermal therapeutic system for the controlled, step-wise administration of active substances to the skin, according to claims 1 to 13, characterized in that, if the plaster is produced in situ, the active substance depot is manufactured by combining depot components.

15. A process according to claims 5, 6, and 7, characterized in that the layers are joined by application of pressure and/or heat.

16. A process according to any one of the preceding claims, characterized in that the active substance depot is inserted into the matrix by the application of pressure.

17. A process according to any one of the preceding claims, characterized in that active substance is incorporated into the matrix during the manufacture of the plaster.

18. A process according to claims 1 to 16, characterized in that the amount of active substance within the matrix is adjusted by diffusion of the active substance from the active substance depot or depots after manufacture of the plaster.

19. A process according to any one of the preceding claims, characterized in that at least one part of the plaster is produced from a solution, a dispersion, a melt or by sprinkling particles thereon.

20. The use of the plaster according to claims 1 to 12 for the local or systemic dermal adminstration of active substances in human or veterinary medicine, or in cosmetics.

## Claims (Claims for the following Contracting State(s): GR, ES)

1. A process for the manufacture of a plaster as transdermal therapeutic system for the controlled and graduated administration of active substances to the skin, with a high initial dosage and lower maintenance dosage, comprising a backing layer averted from the skin, at least one active substance depot in contact with a matrix controlling the active substance release, and a pressure-sensitive adhesive fixing device to secure the plaster to the skin, whereby the complete matrix at the beginning of application comprises active substances, characterized in that the active substance depot or depots in contact with the matrix are dimensioned and positioned in such a way that, at least in one direction, the space between depot and a matrix edge which contacts the releasing surface is larger than the diffusion path of at least one active substance from the depot into the matrix during the course of application, and that the amount of active substance contained in the matrix is, at the time of application, chosen such that the duration of release up to that point in time when the active substance originally contained in the matrix is released is shorter than the duration of application.

2. The process according to claim 1, characterized in that at least one active substance depot is used that exclusively comprises active substance or substances.

3. The process according to claim 1, characterized in that at least one active substance depot is used that comprises at least one inert auxiliary agent.

4. The process according to claims 1 to 3, characterized in that at least one active substance depot is used that is present in solid or flowable form.

5. The process according to any one of the preceding claims, characterized in that the components of the plaster are, at least partially, arranged in layers located one upon the other.

6. The process according to claim 5, characterized in that a matrix is used which consists of at least two layers, whereby preferably at least one active substance depot is incorporated between a back-side matrix layer and a matrix layer facing the skin, and that the thickness ratio of the matrix layers preferably is in the range of 1:1 to 1:20, particularly preferred in the range of 1:1 to 1:5.

7. The process according to claim 5, characterized in that at least one active substance depot is used which is formed in layers and/or in the form of a layer or laminate.

8. The process according to claims 1 to 7, characterized in that a matrix is used that is pressure-sensitive adhesive.

9. The process according to claims 1 to 7, characterized in that the matrix or at least one matrix layer exhibits pressure-sensitive adhesive devices on at least one side.

10. The process according to claims 1 to 7 and 9, characterized in that pressure-sensitive adhesive portions embedded in the matrix are used as fixing devices.

11. The process according to claims 1 to 5 and 7 to 10, characterized in that at least one active substance depot is positioned between matrix and backing layer.

12. The process according to claims 1 to 11, characterized in that a removable protective layer is applied to those surfaces facing the skin.

13. The process according to claims 1 to 12, characterized in that an amount of active substance amounting to up to 20 times the therapeutically required amount is incorporated.

14. A process according to claims 1 to 13, characterized in that, if the plaster is produced in situ, the active substance depot is manufactured by combining depot components.

15. A process according to claims 5, 6, and 7, characterized in that the layers are joined by application of pressure and/or heat.

16. A process according to any one of the preceding claims, characterized in that the active substance depot is inserted into the matrix by the application of pressure.

17. A process according to any one of the preceding claims, characterized in that active substance is incorporated into the matrix during the manufacture of the plaster.

18. A process according to claims 1 to 16, characterized in that the amount of active substance within the matrix is adjusted by diffusion of the active substance from the active substance depot or depots after manufacture of the plaster.

19. A process according to any one of the preceding claims, characterized in that at least one part of the plaster is produced from a solution, a dispersion, a melt or by sprinkling particles thereon.

20. The use of the plaster according to claims 1 to 19 for the local or systemic dermal adminstration of active substances in cosmetics.

## Revendications (Revendications pour l'(les) Etat(s) contractant(s) suivant(s): AT, BE, CH, DE, DK, FR, GB, IT, LI, LU, NL, SE)

1. Timbre servant de système thérapeutique transdermique pour l'administration régulée et échelonnée de principes actifs ou substances actives à la peau, à dose initiale plus élevée et à dose d'entretien plus faible, qui comprend une couche dorsale écartée de la peau, au moins un dépôt de principe actif en contact avec une matrice régulant la libération du principe actif et un dispositif de fixation autocollant pour le timbre sur la peau, où au début de l'application, la matrice totale contient du principe actif, qui se caractérise en ce que l'on choisit les dimensions et la position du ou des dépôts de principe actif en contact avec la matrice en une manière telle qu'au moins, dans une direction, la distance entre dépôt et un bord de la matrice touchant la surface de libération soit supérieure au trajet de diffusion d'au moins un principe actif à partir du dépôt dans la matrice au cours de la durée de l'application et que l'ampleur de la teneur en principe actif de la matrice au moment de l'application soit choisie de façon que la durée de libération jusqu'à la libération du principe actif contenu à l'origine dans la matrice soit plus brève que la durée de l'application.

2. Timbre suivant la revendication 1, caractérisé en ce qu'au moins un dépôt de principe actif présente exclusivement un ou des principes actifs.

3. Timbre suivant la revendication 1, caractérisé en ce qu'au moins un dépôt de principe actif présente au moins un adjuvant.

4. Timbre suivant l'une quelconque des revendications 1 à 3, caractérisé en ce qu'au moins un dépôt de principe actif se présente sous une forme solide ou fluide.

5. Timbre suivant l'une quelconque des revendications précédentes, caractérisé en ce qu'il est constitué au moins partiellement sous forme stratifiée et/ou sous forme de couche.

6. Timbre suivant la revendication 5, caractérisé en ce que la matrice se compose d'au moins deux couches, où au moins un dépôt de principe actif est mis en place, de préférence, entre une couche de matrice dorsale et une couche de matrice faisant face à la peau et le rapport des épaisseurs des couches de matrice se situe, de préférence, dans la plage de 1:1 à 1:20 et, de manière particulièrement avantageuse, dans la plage de 1:1 à 1:5.

7. Timbre suivant la revendication 1, caractérisé en ce qu'au moins un dépôt de principe actif est agencé sous forme stratifiée et/ou sous forme de couche.

8. Timbre suivant l'une quelconque des revendications précédentes, caractérisé en ce que la matrice est autocollante

9. Timbre suivant l'une quelconque des revendications 1 à 7, caractérisé en ce que la matrice, ou au moins une couche de matrice, présente des dispositifs au moins autocollants d'un côté.

10. Timbre suivant l'une quelconque des revendications 1 à 7 et 9, caractérisé en ce que le dispositif de fixation dans la matrice est constitué par des découpes de colle de contact noyées.

11. Timbre suivant l'une quelconque des revendications 1 à 5 et 7 à 10, caractérisé en ce qu'au moins un dépôt de principe actif est agencé entre la couche de matrice et la couche dorsale.

12. Timbre suivant l'une quelconque des revendications précédentes, caractérisé en ce qu'il présente une couche de protection amovible pour les surfaces qui font face à la peau.

13. Timbre suivant l'une quelconque des revendications précédentes, caractérisé en ce qu'il contient jusqu'à 20 fois la quantité de principe actif thérapeutiquement nécessaire.

14. Procédé de fabrication d'un timbre comme système thérapeutique transdermique pour l'administration réglée et échelonnée de principes actifs à la peau suivant l'une quelconque des revendications 1 à 13, caractérisé en ce que l'on réalise le dépôt de principe actif in situ au cours de la fabrication du timbre par la réunion des composants du dépôt.

15. Procédé suivant l'une quelconque des revendications 5, 6 et 7, caractérisé en ce que les couches sont assemblées sous pression et/ou sous l'effet de la chaleur.

16. Procédé suivant l'une quelconque des revendications précédentes, caractérisé en ce que le dépôt de principe actif est incorporé à la matrice sous pression.

17. Procédé suivant l'une quelconque des revendications précédentes, caractérisé en ce que le principe actif est incorporé à la matrice au cours de la fabrication du timbre.

18. Procédé suivant l'une quelconque des revendications 1 à 16, caractérisé en ce que la teneur en principe actif de la matrice est réglée, après la fabrication du timbre, par diffusion de principe actif à partir du ou des dépôts de principe actif.

19. Procédé suivant l'une quelconque des revendications précédentes, caractérisé en ce qu'au moins une partie du timbre est fabriquée à partir d'une solution, d'une dispersion, d'une masse fondue ou par saupoudrage ou épandage de particules.

20. Utilisation du timbre suivant l'une quelconque des revendications 1 à 12, en vue de l'administration dermique de principe actif, locale ou systémique dans le domaine cosmétique.

## Revendications (Revendications pour l'(les) Etat(s) contractant(s) suivant(s): GR, ES)

1. Procédé de fabrication d'un timbre servant de système thérapeutique transdermique pour l'administration régulée et échelonnée de principes actifs ou substances actives à la peau, à dose initiale plus élevée et à dose d'entretien plus faible, qui comprend une couche dorsale écartée de la peau, au moins un dépôt de principe actif en contact avec une matrice régulant la libération du principe actif et un dispositif de fixation autocollant pour le timbre sur la peau, où au début de l'application, la matrice totale contient du principe actif, caractérisé en ce que l'on dimensionne et agence le dépôt de principe actif en contact avec la matrice en une manière telle qu'au moins, dans une direction, la distance entre dépôt et un bord de la matrice touchant la surface de libération soit supérieure au trajet de diffusion d'au moins un principe actif à partir du dépôt dans la matrice au cours de la durée de l'application et que l'ampleur de la teneur en principe actif de la matrice au moment de l'application soit choisie de façon que la durée de libération jusqu'à la libération du principe actif contenu à l'origine dans la matrice soit plus brève que la durée de l'application.

2. Procédé suivant la revendication 1, caractérisé en ce que l'on utilise au moins un dépôt de principe actif qui contient exclusivement un ou des principes actifs.

3. Procédé suivant la revendication 1, caractérisé en ce que l'on utilise au moins un dépôt de principe actif qui contient au moins un adjuvant inerte.

4. Procédé suivant l'une quelconque des revendications 1 à 3, caractérisé en ce que l'on utilise au moins un dépôt de principe actif qui se présente sous forme solide ou fluide.

5. Procédé suivant l'une quelconque des revendications précédentes, caractérisé en ce que ses constituants sont mutuellement superposés au moins en partie sous forme de couche.

6. Procédé suivant la revendication 5, caractérisé en ce que l'on utilise une matrice qui se compose d'au moins deux couches, où au moins un dépôt de principe actif est mis en place, de préférence, entre une couche de matrice dorsale et une couche de matrice faisant face à la peau et le rapport des épaisseurs des couches de matrice se situe, de préférence, dans la plage de 1:1 à 1:20 et, de manière particulièrement avantageuse, dans la plage de 1:1 à 1:5.

7. Procédé suivant la revendication 5, caractérisé en ce que l'on utilise au moins un dépôt de principe actif réalisé sous forme stratifiée et/ou sous forme de couche.

8. Procédé suivant l'une quelconque des revendications précédentes, caractérisé en ce que l'on utilise une matrice autocollante

9. Procédé suivant l'une quelconque des revendications 1 à 7, caractérisé en ce que la matrice utilisée, ou au moins l'une de ses couches, présente des dispositifs au moins autocollants d'un côté.

10. Procédé suivant l'une quelconque des revendications 1 à 7 et 9, caractérisé en ce que, à titre de dispositif de fixation dans la matrice on utilise des découpes de colle de contact noyées.

11. Procédé suivant l'une quelconque des revendications 1 à 5 et 7 à 10, caractérisé en ce qu'au moins un dépôt de principe actif est agencé entre la couche de matrice et la couche dorsale.

12. Procédé suivant l'une quelconque des revendications 1 à 11, caractérisé en ce que l'on applique une couche de protection amovible sur les surfaces qui font face à la peau.

13. Procédé suivant l'une quelconque des revendications 1 à 12, caractérisé en ce que l'on incorpore jusqu'à 20 fois la quantité de principe actif thérapeutiquement nécessaire.

14. Procédé suivant l'une quelconque des revendications 1 à 13, caractérisé en ce que l'on réalise le dépôt de principe actif in situ au cours de la fabrication du timbre par la réunion des composants du dépôt.

15. Procédé suivant l'une quelconque des revendications 5, 6 et 7, caractérisé en ce que les couches sont assemblées sous pression et/ou sous l'effet de la chaleur.

16. Procédé suivant l'une quelconque des revendications précédentes, caractérisé en ce que le dépôt de principe actif est incorporé à la matrice sous pression.

17. Procédé suivant l'une quelconque des revendications précédentes, caractérisé en ce que le principe actif est incorporé à la matrice au cours de la fabrication du timbre.

18. Procédé suivant l'une quelconque des revendications 1 à 16, caractérisé en ce que la teneur en principe actif de la matrice est réglée, après la fabrication du timbre, par diffusion de principe actif à partir du ou des dépôts de principe actif.

19. Procédé suivant l'une quelconque des revendications précédentes, caractérisé en ce qu'au moins une partie du timbre est fabriquée à partir d'une solution, d'une dispersion, d'une masse fondue ou par saupoudrage ou épandage de particules.

20. Utilisation du timbre suivant l'une quelconque des revendications 1 à 19, en vue de l'administration dermique de principe actif, locale ou systémique dans le domaine cosmétique.
